# EUROPEAN PATENT SPECIFICATION

(11) **EP 3 990 075 B1**
(45) Date of publication and mention of the grant of the patent: **15.10.2025**
(21) Application number: 19825304.9
(22) Date of filing: 25.06.2019
(51) Int. Cl.: A61M 16/06, A61M 16/04, A61B 1/267, A61M 16/20, A61M 16/08

(54) **VENTILATED INTUBATION APPARATUS**
GERÄT ZUR BEATMUNGSINTUBATION
APPAREIL D'INTUBATION VENTILÉ

(43) Date of publication of application: 04.05.2022
(73) Proprietor: AIRWAY MEDICAL INNOVATIONS PTY LTD, Fortitude Valley, QLD 4006 (AU)
(72) Inventor: ALONSO BABARRO, Julio Miguel, Fortitude Valley, Queensland 4006 (AU)
(74) Representative: Regimbeau
(86) International application number: PCT/AU2019/050652
(87) International publication number: WO 2020/000031

(56) References cited:
- EP-A2- 3 150 245
- GB-A- 2 332 375
- US-A- 5 197 463
- US-A- 5 694 929
- US-A- 5 694 929
- US-A1- 2009 211 574
- US-A1- 2014 332 000
- US-B2- 6 568 388
- US-B2- 6 763 831
- US-B2- 8 960 195
- US-B2- 8 960 195

## Description

### Background of the Invention

The present technology relates to a method and apparatus for use in an endotracheal intubation procedure, being particularly adapted to allow ventilation of a subject throughout the procedure.

### Description of the Prior Art

Endotracheal intubation is the procedure through which a medical professional introduces a flexible plastic conduit, an endotracheal tube, generally through the mouth and into the trachea. This allows artificial ventilation, which is required when the breathing ability is compromised by an illness or injury in an emergency situation or is interfered by drug-induced depression during surgery. It is a universal procedure and is performed in the same fashion all over the world.

Every day thousands of intubations are performed by a diverse range of professionals, particularly anaesthetics specialists, intensivists, emergency physicians and pre-hospital medics and paramedics. However endotracheal intubation is a high risk procedure which can lead to death or disability, requires considerable skill and occasionally cannot be accomplished. Even to highly trained professionals, it is often difficult and sometimes unsuccessful. New specialised instruments and advanced techniques are continuously developing with the aim to facilitate this difficult procedure and ensure better success rates.

The aim of the operator is to successfully pass an endotracheal tube through the mouth, pharynx and larynx and into the trachea. The oropharyngeal passage is curved and narrow and ends at the entrance of both the larynx and the oesophagus. The tongue tends to fall back on to the pharynx when a patient is in supine position, the entrance of the larynx can vary in its position due to the particular anatomy of a patient and the epiglottis lies over the entrance of the larynx and usually needs to be moved to expose the glottic opening.

The operator needs to identify the vocal cords at the entrance of the larynx, the epiglottis above the entrance of the larynx in the transversal view with the patient supine, and the oesophagus, below all previous structures on this view. This procedure requires extraordinary skills; it is easier for the endotracheal tube to follow the path towards the oesophagus, it is often difficult to obtain a good view of the larynx, and even with a good view, it is difficult sometimes to introduce the endotracheal tube. Any delay in successfully finalising the procedure is a serious complication, and may potentially be fatal.

The insertion of an endotracheal tube through all these anatomical structures and into the trachea is referred to as endotracheal intubation and typically requires the use of an instrument called laryngoscope which comprises a handle, and a blade. Different shapes of the blade may be used depending on a range of factors such as the age or size of the patient and different procedural options. Laryngoscope blades are generally classified as curved or straight, although a number of styles of curved and straight blades are commercially available. Some styles of blades are designed to be positioned anterior to the epiglottis, and other styles are designed to be positioned posterior to the epiglottis, leading to slightly different movements during the procedure.

During endotracheal intubation, generally with the patient laying supine, the operator, standing at the top of the head of the patient, introduces the blade of the laryngoscope through the mouth and into the pharynx and manipulates anatomical structures such as the tongue and the epiglottis (depending on the particular patient and type of blade) to expose the entrance of the larynx. Then, under direct visualisation, the operator inserts the tip of the endotracheal tube into the larynx and advances it into the trachea. In the conventional and universal procedure, the operator typically utilises the left hand to hold the laryngoscope by the handle to position the blade and utilises the right hand to carefully introduce the endotracheal tube, pushing it alongside the laryngoscope blade and into the visualised trachea.

Often, because of anatomical variations and challenges, and despite an adequate technique, direct visualisation is difficult. In most of these occasions, adequate visualisation could be obtained by manipulating some of the anatomical structures. Unfortunately, with the conventional laryngoscope and conventional procedure, the operator is utilising both hands and the hand being used to manually introduce the endotracheal tube cannot be used to manipulate anatomical structures to facilitate the procedure. Furthermore, a second operator could not have direct visual access to the entrance of the larynx to help manipulating these structures and will interfere with the vision of the first operator is obtaining the view, is very limited and the operator performing the intubation procedure will usually be in the best viewing position. Video laryngoscopes are available to remove the need for a direct view, although these are typically bulkier than conventional laryngoscopes and still occupy both hands of the operator.

Due to the degree of difficulty of the procedure itself, together with the seriousness of the potential complications, this procedure will only be performed by highly skilled professionals. This difficulty and serious complication risk have also meant that the procedure, and the instruments used to perform it, has essentially remained unchanged for decades. The physicians and other professionals who perform endotracheal intubations are unwilling to use new devices or to change the way this is conventionally done, given the difficulties and risks. A new intubation device therefore not only has to offer obvious procedural advantages in comparison to the conventional laryngoscopes, but also has to present similar characteristics in shape and weight and in its method of use, to facilitate adoption by operators already trained and comfortable in the use of conventional laryngoscopes in the often stressful circumstances of performing an intubation procedure.
Patent application US5697929 discloses a method and apparatus for ventilation/oxygenation during guided insertion of an endotracheal tube. Patent US8960195 discloses an intubation-facilitating oxygen mask.

WO/2016/090435A1 discloses a new intubation device that allows an endotracheal intubation procedure to be performed using a single hand. In particular, the intubation device includes: a laryngoscope blade having a tip and a base; a handle attached to the base of the blade for allowing the intubation device to be held in a hand of a user; a channel for receiving an endotracheal tube, the channel including a blade channel portion extending along the blade substantially from the tip to the base and including an outlet proximate to the tip for allowing a distal end of the endotracheal tube to be advanced from the outlet and a handle channel portion extending partially along the handle from the blade channel portion; and a tube movement mechanism in the handle for moving the endotracheal tube through the channel to thereby advance the endotracheal tube, the tube movement mechanism including a thumb interface for allowing the user to operate the tube movement mechanism using a thumb of the hand that is holding the intubation device, to thereby allow the user to hold the intubation device and advance the endotracheal tube in an endotracheal intubation procedure using a single hand.

By enabling single handed operation of the intubation device for positioning the blade via the handle and advancing the endotracheal tube, the other hand of the user will remain free for other uses, such as clearing the airway using another device, such as a suction device, or other devices such as forceps or the like to manipulate anatomical structures and/or the endotracheal tube, during the endotracheal intubation procedure as may be required.

Whilst such a single handed intubation device can help to significantly reduce the difficulty and serious complication risk of endotracheal intubation procedure, one remaining problem is that there will be a period of time during the procedure in which artificial ventilation cannot be provided to the patient. Typically, a patient will be ventilated by mask prior to the procedure until a sufficiently high blood oxygen saturation level is achieved. Typically this may involve pre-oxygenation to reach a 100% blood oxygen saturation, and optionally sedation or paralysis of the patient. When the desired blood oxygen saturation level is achieved, ventilation is discontinued and the mask is removed to expose the patient's mouth and allow the endotracheal intubation procedure to be performed. During the procedure, the patient will not be ventilated and it is therefore vital that the endotracheal intubation is completed as quickly as possible so that ventilation can be resumed via the endotracheal tube. If complications arise during the intubation procedure, there can be a significant risk associated with interrupting ventilation for an excessive period of time. This risk can be exacerbated in situations where the patient is severely ill or injured before the intubation procedure, making ventilation even more critical. It would be therefore be desirable to provide a method and apparatus for allowing an endotracheal intubation procedure to be performed without interrupting ventilation.

US5964217A discloses a method and apparatus for ventilation/oxygenation during guided insertion of an endotracheal tube. An endotracheal tube can be inserted into a patient's trachea during resuscitation by using a face mask and a curved guide. The guide is inserted through a flexible port in the face mask and has a curved distal portion that extends into the patient's mouth and hypopharynx. The patient is initially resuscitated by supplying a flow of air/oxygen through the mask. An endotracheal tube is inserted over the distal end of a fiber optic probe. Resuscitation, oxygenation, or artificial ventilation continue without interruption while the fiber optic probe and endotracheal tube are inserted through a flexible port at the proximal end of the curve guide and then advanced along the guide into the patient's airway. The direction of the distal tip of the fiber optic probe can be controlled by the physician. This allows the physician to carefully guide the fiber optic probe and endotracheal tube to a position past the larynx while resuscitation continues. The fiber optic probe is then removed from within the endotracheal tube and the mask is removed while leaving the endotracheal tube in place within the trachea. The cuff on the endotracheal tube is inflated and a ventilator is connected to the proximal end of the endotracheal tube to ventilate the patient. Alternatively, the patient can be manually ventilated by connecting a resuscitation bag to the proximal end of the endotracheal tube.

However, such an apparatus will only be useful in relatively straightforward intubations which would not require a laryngoscope or intubation device as discussed above. There is a need for an improved method and apparatus to allow ventilated intubation to be performed under a wider range of conditions more likely to be encountered in practice, ideally using an intubation device similar to those already routinely used by medical practitioners.

The reference in this specification to any prior publication (or information derived from it), or to any matter which is known, is not, and should not be taken as an acknowledgment or admission or any form of suggestion that the prior publication (or information derived from it) or known matter forms part of the common general knowledge in the field of endeavour to which this specification relates.

### Summary of the Present disclosure

The invention is defined by the appended claims.

In one broad form an aspect of the present technology seeks to provide a method for use in an endotracheal intubation procedure, the method including: inserting an intubation guide into a mouth of a subject, the intubation guide including an elongate body defining a passageway extending between a proximal opening and a distal opening, the passageway being configured for receiving a blade portion of an intubation device, and the proximal opening being positioned proximate to the mouth of the subject and the distal opening being positioned proximate to a pharynx of the subject; covering the mouth of the subject with a ventilation mask including: a ventilation port connected to a ventilator; and an intubation port for receiving the blade portion of an intubation device, the intubation port being aligned with the mouth of the subject; ventilating the subject using the ventilation mask; and while ventilation of the subject continues: inserting the blade portion of the intubation device into the passageway of the intubation guide through the intubation port; positioning a distal tip of the blade portion of the intubation device proximate to the larynx of the subject; and advancing an endotracheal tube along the blade portion of the intubation device through the intubation port and the intubation guide into a trachea of the subject.

In one embodiment, the method includes, after advancing the endotracheal tube into the trachea of the subject, and while leaving the endotracheal tube in place in the trachea of the subject: withdrawing the blade portion of the intubation device from the intubation guide and the intubation port; removing the ventilation mask from the subject; and removing the intubation guide from the mouth of the subject.

In one embodiment, at least one of: the intubation guide is configured to be broken along the passageway, the method including breaking the intubation guide to allow the intubation guide to be removed while the endotracheal tube remains in place; and the ventilation mask is configured to be broken about the intubation port, the method including breaking the ventilation mask to allow the ventilation mask to be removed while the endotracheal tube remains in place.

In one embodiment, the ventilation mask includes a closure for covering the intubation port, the method including removing the closure prior to inserting the blade portion through the intubation port.

In one embodiment, the ventilation mask includes a seal covering the intubation port, the seal normally being in a closed position for sealing the intubation port and being moveable to an open position when the blade portion of the intubation device is inserted through the intubation port, the method including inserting the blade portion through the seal of the intubation port.

In one embodiment, ventilating the subject using the ventilation mask includes oxygenating the subject using the ventilation mask.

In another broad form an aspect of the present technology seeks to provide apparatus for use in an endotracheal intubation procedure, the apparatus including: an intubation guide including an elongate body defining a passageway extending between a proximal opening and a distal opening, the passageway being configured for receiving a blade portion of an intubation device, and the intubation guide being configured for insertion into a mouth of the subject so that the proximal opening is positioned proximate to the mouth of the subject and the distal opening is positioned proximate to a pharynx of the subject; and a ventilation mask for covering the mouth of the subject, the ventilation mask including: a ventilation port for connection to a ventilator; and an intubation port for receiving the blade portion of the intubation device, the intubation port being configured to align with the mouth of the subject to thereby allow the blade portion to be inserted into the passageway of the intubation guide through the intubation port.

In one embodiment, the ventilation mask includes a closure for covering the intubation port when the blade portion is not inserted through the intubation port.

In one embodiment, the ventilation mask includes a seal covering the intubation port, the seal normally being in a closed position for sealing the intubation port and being moveable to an open position when the blade portion of the intubation device is inserted through the intubation port.

In one embodiment, the seal includes at least one resilient membrane configured to deform in response to the blade portion being urged against the seal, to thereby define an opening for receiving the blade portion.

In one embodiment, the seal includes a resilient membrane that is supported around a perimeter of the intubation port, the resilient membrane including an aperture that is substantially closed in the closed position and that stretches to define the opening in the open position.

In one embodiment, the aperture is a slit.

In one embodiment, the seal includes two or more resilient membranes that are supported around the perimeter of the intubation port, the respective aperture of each resilient membrane differing from the apertures of other resilient membranes in at least one of: shape; location; and orientation.

In one embodiment, the seal includes two or more resilient membranes that are each supported around a respective part of a perimeter of the intubation port and that each include a respective unsupported edge, the unsupported edges at least partially overlapping in the closed position and separating to define the opening in the open position.

In one embodiment, the ventilation mask is for covering the mouth and a nose of the subject.

In one embodiment, the ventilation port is located proximate to the nose covering portion of the ventilation mask.

In one embodiment, the ventilation port is configured to direct a flow of ventilation gas towards nostrils of the subject.

In one embodiment, the ventilation mask includes an internal partition for defining separate volumes proximate to the mouth and the nose of the subject.

In one embodiment, the ventilation port is offset laterally relative to a central plane of the ventilation mask that is aligned with a sagittal plane of the subject when the ventilation mask covers the mouth of the subject.

In one embodiment, the ventilation mask is configured to be broken about the intubation port.

In one embodiment, the intubation guide is separate from the ventilation mask and moveable relative to the ventilation mask in use.

In one embodiment, the intubation guide includes a flange surrounding the proximal opening.

In one embodiment, the flange is configured to prevent over-insertion of the intubation guide by abutting the subject's mouth to thereby ensure that the proximal opening remains positioned outside the mouth.

In one embodiment, the intubation guide is configured to be broken along the passageway.

In one embodiment, the body of the intubation guide includes two break lines extending longitudinally along opposing sides of the passageway, to thereby allow the intubation guide to be broken along the break lines.

In one embodiment, the break lines are defined along a central plane of the intubation guide.

In one embodiment, the intubation port of the ventilation mask and the passageway of the intubation guide have similar cross sections.

In one embodiment, a shape of the intubation port is selected based on a cross section shape of the blade portion of the intubation device.

In one embodiment, a size of the intubation port is smaller than a cross section size of the blade portion of the intubation device.

In one embodiment, a shape of the passageway is selected based on a cross section shape of the blade portion of the intubation device.

In one embodiment, a size of the passageway is smaller than a cross section size of the blade portion of the intubation device.

In one embodiment, at least one of the intubation guide and the ventilation mask is formed from a flexible material.

In one embodiment, at least one of the intubation guide and the intubation port is configured to expand when receiving the blade portion.

In one embodiment, the intubation guide is curved.

In one embodiment, a curvature of the intubation guide is selected based on a curvature of the blade portion of the intubation device.

In one embodiment, the intubation guide is configured to, in use, at least one of: hold a tongue of the subject; and depress the tongue.

In another broad form an aspect of the present technology seeks to provide an intubation guide for use in an endotracheal intubation procedure, the intubation guide including an elongate body defining a passageway extending between a proximal opening and a distal opening, the passageway being configured for receiving a blade portion of an intubation device, and the intubation guide being for insertion into a mouth of the subject so that the proximal opening is positioned proximate to the mouth and the distal opening is positioned proximate to a pharynx of the subject.

In another broad form an aspect of the present technology seeks to provide a ventilation mask for use in an endotracheal intubation procedure, the ventilation mask being for covering a mouth of the subject, the ventilation mask including: a ventilation port for connection to a ventilator; and an intubation port for receiving a blade portion of an intubation device, the intubation port being configured to align with a mouth of the subject to thereby allow the blade portion to be inserted into the mouth through the intubation port.

It will be appreciated that the broad forms of the technology and their respective features can be used in conjunction, interchangeably and/or independently, and reference to separate broad forms is not intended to be limiting.

### Brief Description of the Drawings

Various examples and embodiments of the present disclosure will now be described with reference to the accompanying drawings, in which: -
Figures 1A to 1D are cross section views showing steps of a ventilated endotracheal intubation procedure being performed on a subject;
Figure 2 is a perspective view of an example of a ventilation mask connected to a ventilator for use in the ventilated endotracheal intubation procedure;
Figures 3A and 3B are perspective views of an example of the ventilation mask of Figure 2;
Figures 4A and 4B are perspective views of a blade portion of an intubation device being received in an intubation port of the ventilation mask of Figures 3A and 3B;
Figures 5A to 5C are perspective views of an example of an intubation guide for use in the ventilated endotracheal intubation procedure; and
Figures 6A and 6B are perspective views of a blade portion of an intubation device being received in the intubation guide of Figures 5A to 5C.

### Detailed Description of the Preferred Embodiments

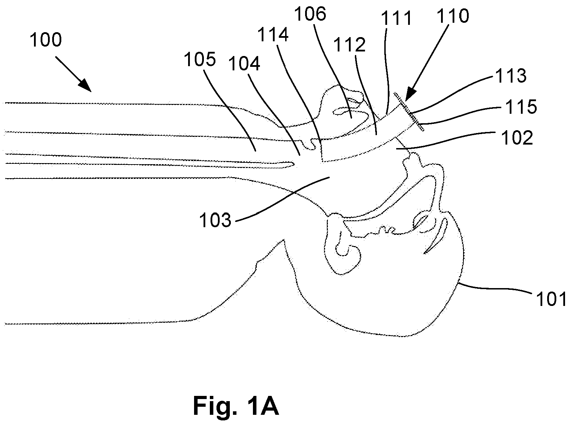

An example of a method for use in a ventilated endotracheal intubation procedure will now be described with reference to Figures 1A to 1D. The method involves the use of an apparatus including an intubation guide 110 and a ventilation mask 120, both particularly adapted to allow intubation to be performed using a bladed intubation device, such as a laryngoscope or the like. For the purpose of the following examples, it is assumed that the intubation device 140 is a single handed intubation device as described in WO/2016/090435A1, although it should be appreciated that other forms of intubation devices may be used with appropriate adaptations to the apparatus.

With reference to Figure 1A, the method commences with the subject 100 lying in a supine position, in a similar manner as per a conventional endotracheal intubation procedure. The subject's head 101 may be tilted, to adjust the relative positioning of the subject's mouth 102, pharynx 103 and larynx 104 to better facilitate access to the larynx during the procedure.

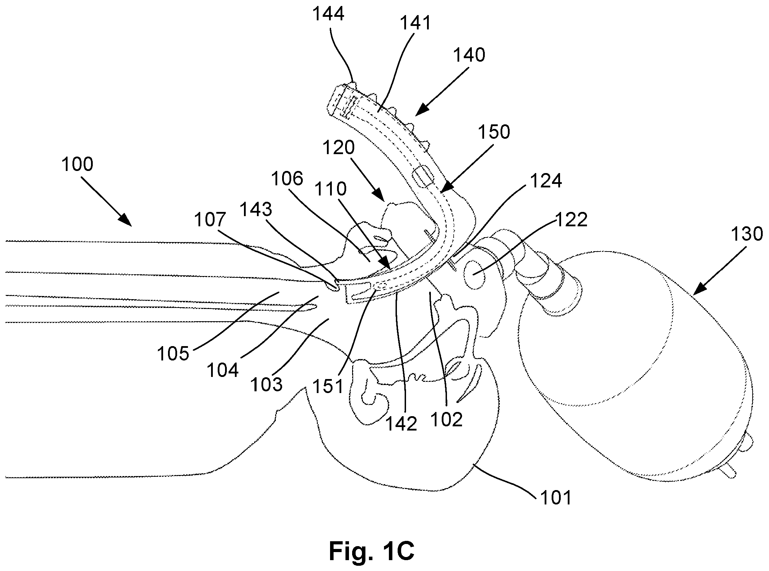

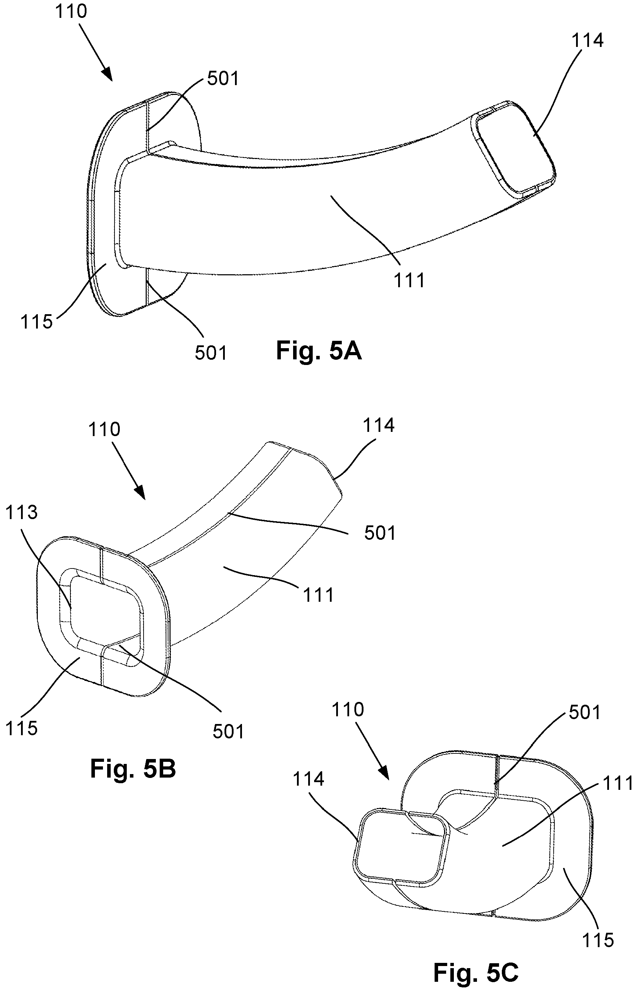

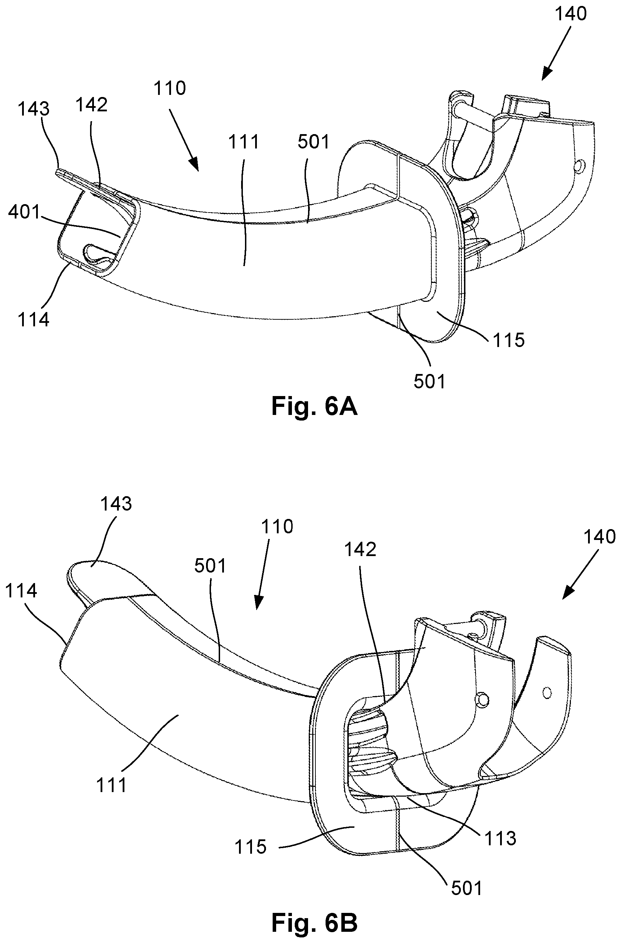

The intubation guide 110 is then inserted into the mouth 102 of the subject 100. Further details of the intubation guide 110 can be seen in Figures 5A to 5C. The intubation guide 110 includes an elongate body 111 defining a passageway extending between a proximal opening 113 and a distal opening 114. The passageway of the intubation guide 110 is configured for receiving a blade portion 142 of an intubation device 140, as shown in Figure 1C and in further detail in Figures 6A and 6B. After the intubation guide 110 has been inserted, the proximal opening 113 is positioned proximate to the mouth 102 of the subject 100 and the distal opening 114 is positioned proximate to a pharynx 103 of the subject 100.

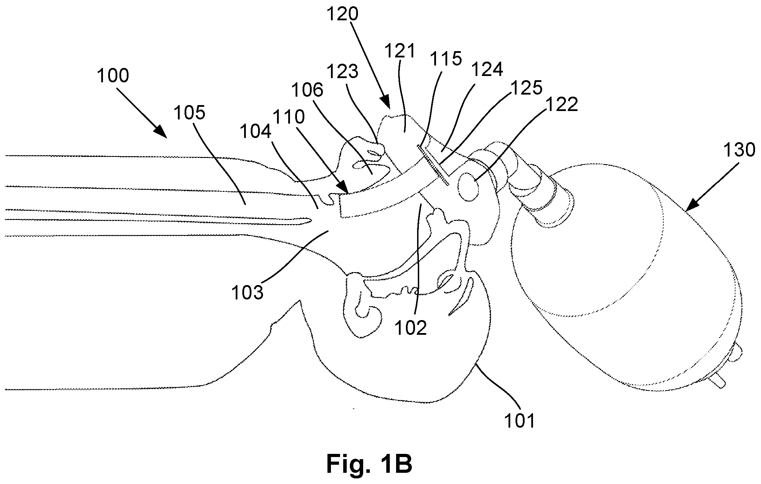

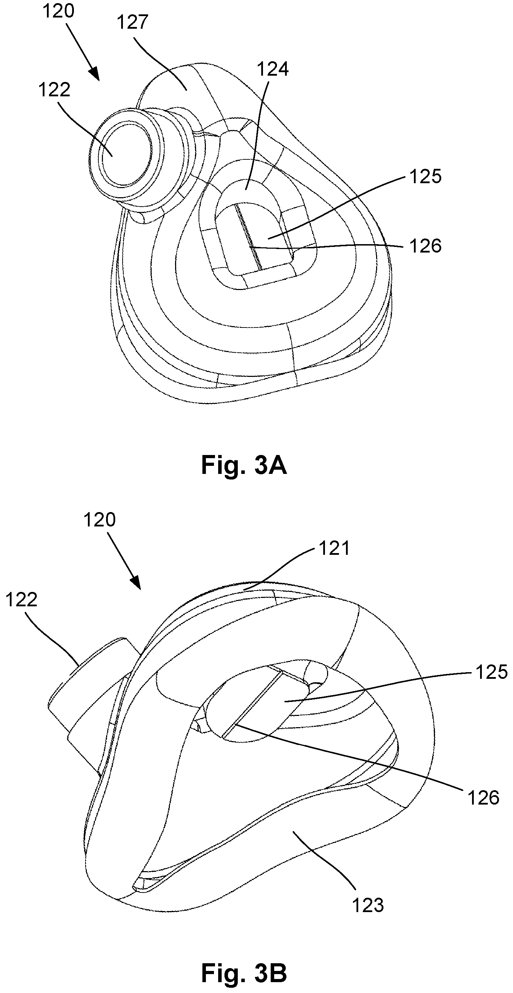

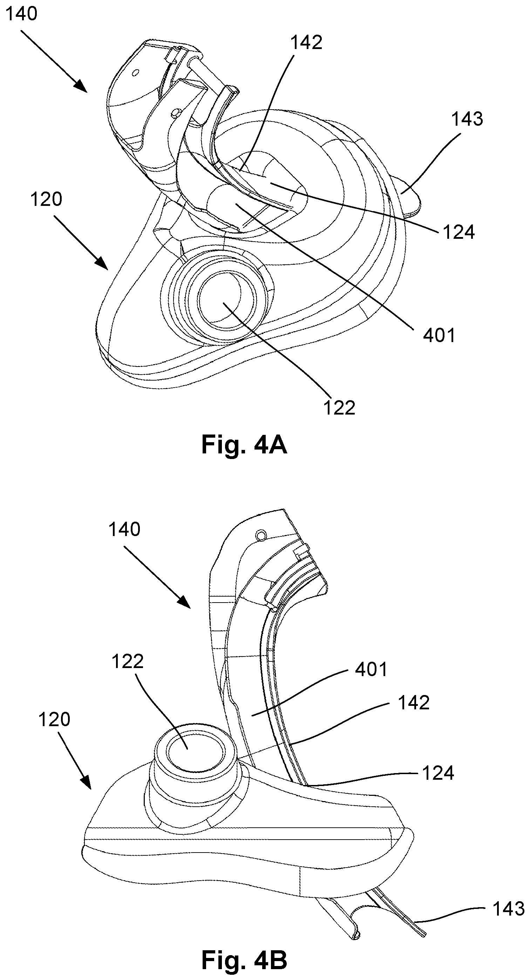

Turning to Figure 1B, the mouth 102 of the subject 100 is then covered with the ventilation mask 120. Further details of the ventilation mask 110 can be seen in Figures 3A and 3B. The ventilation mask 120 includes a ventilation port 122 connected to a ventilator 130, an intubation port 124. The intubation port 124 is particularly configured for receiving the blade portion 142 of the intubation device 140, as shown in Figure 1C and in further detail in Figures 4A and 4B. The ventilation mask 120 is configured so that the intubation port 124 is aligned with the mouth 102 of the subject 100.

Once the subject's mouth 102 is covered by the ventilation mask 120, the subject 100 may be ventilated using the ventilation mask 120 and the connected ventilator 130. It should be appreciated that any suitable type of ventilator 130 may be used for ventilating the subject 100. Whilst the depicted examples show a ventilator 130 in the form of a manual bag-valve mask ventilator connected to the ventilation mask 120, the ventilator may alternatively be in the form of a powered mechanical ventilator, for example.

The process of ventilating the subject using the ventilation mask may include oxygenating the subject using the ventilation mask, such as by supplying oxygen gas from an oxygenation source to the ventilation port. In some cases, 100% oxygen may be supplied or air may be supplied at lower oxygen percentages. Nevertheless, it should be appreciated that oxygenation is not essential and the ventilation may be provided using atmospheric air without added oxygen. This may depend on whether a separate pressurised oxygen source is available, which may not be the case in some circumstances.

With regard to Figure 1C, while ventilation of the subject 100 continues, the blade portion 142 of the intubation device 140 is inserted into the passageway 112 of the intubation guide 110, through the intubation port 124 of the ventilation mask 120.

The blade portion 142 of the intubation device 140 is passed through the passageway 112 of the intubation guide 110 to position a distal tip 143 of the blade portion 142 proximate to the larynx 104 of the subject 100. The specific positioning of the distal tip 143 will depend of the particular configuration of the blade, but typically the distal tip 143 will be positioned around the epiglottis 107 and moved as required to expose the glottis. As the blade portion 142 is moved, this may result in some movement of the intubation guide 110 relative to the subject's mouth 102 and tongue 106.

As mentioned, above, in this example the intubation device 140 is a single handed intubation device and includes a handle portion 141 connected to the blade portion 142, for allowing the user to hold the intubation device 140 and move the blade portion 142 and the distal tip 143 relative to the subject's anatomy. This form of intubation device 140 includes a channel for receiving an endotracheal tube 150, and a tube movement mechanism in the handle portion 141 for moving the endotracheal tube 150 through the channel to thereby advance the endotracheal tube 150. In this case, the tube movement mechanism includes a thumb interface 144 for allowing the user to operate the tube movement mechanism using a thumb of the hand that is holding the intubation device, to thereby allow the user to hold the intubation device 140 and advance the endotracheal tube 150 in an endotracheal intubation procedure using a single hand.

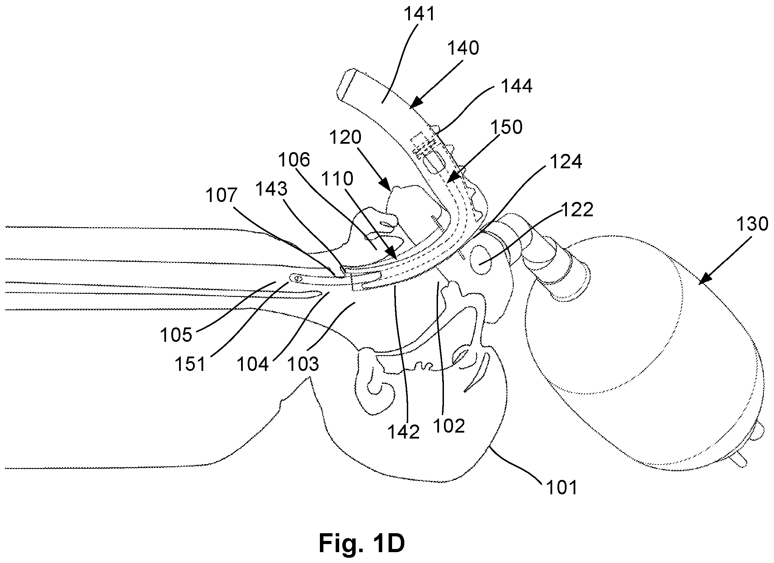

Now turning to Figure 1D, once the distal tip 143 has been moved to an appropriate position to allow endotracheal intubation, the endotracheal tube 150 is advanced along the blade portion 142 of the intubation device 140 through the intubation port 124 of the ventilation mask 120 and the intubation guide 110, into the trachea 105 of the subject. It will be appreciated that the use of a single handed intubation device 140 allows the endotracheal tube 150 to be advanced by the user operating the thumb interface 144 using the thumb of the same hand holding the device. However, if a different form of intubation device that does not facilitate single handed intubation is used, the endotracheal tube 150 may be advanced manually, in a generally conventional manner.

In any event, as mentioned above, the entire endotracheal intubation procedure including inserting the intubation device 140 and advancing the endotracheal tube 150 into the trachea 105 may be performed while the subject 100 is ventilated, thereby avoiding the potentially risky period without ventilation that takes place in conventional endotracheal intubation procedures. Accordingly, the method may be used to allow endotracheal intubation to be performed without the time pressure that medical professionals often face in conventional endotracheal intubation procedures. It will be appreciated that this can greatly improve the likelihood of successful intubation, even in traditionally difficult circumstances.

Once the endotracheal tube 150 is in position with the trachea 105 of the subject, the endotracheal tube 150 can be coupled to a ventilation source and used to provide ventilation to the subject. The ventilation source may be the same as that used to provide ventilation using the ventilation mask 120, or may be a different ventilation source. It is preferable that ventilation using the endotracheal tube 150 is established before or immediately after ceasing ventilation using the ventilation mask 120, so as to ensure that the subject is continuously ventilated.

In some examples, it may be desirable to confirm effective ventilation using the endotracheal tube 120 before removing the ventilation mask 120, and if this is not the case, ventilation may be continued using the ventilation mask 120 while the user repositions the endotracheal tube 150 to allow effective ventilation to be achieved.

After the endotracheal tube 150 has been advanced into the trachea 105 of the subject 100, the intubation device 140 and apparatus may be removed while leaving the endotracheal tube 150 in place in the trachea 105. This may be performed by withdrawing the blade portion 142 of the intubation device 140 from the intubation guide 110 and the intubation port 124, then removing the ventilation mask 120 from the subject 100, and finally removing the intubation guide 110 from the mouth 102 of the subject 100. As mentioned above, ventilation using the endotracheal tube 150 should commence before or immediately after the ventilation mask 120 is removed from the subject 100, such that ventilation using the ventilation mask 120 is no longer required.

In some embodiments, the intubation guide 110 may be configured to be broken along the passageway 112. For instance, with regard to the example intubation guide 110 shown in Figures 5A to 5C, this may be facilitated by defining break lines 501 in the body 111, further details of which will be described in due course. In any event, when using such an intubation guide 110, the method may include breaking the intubation guide 110 to allow the intubation guide 110 to be removed while the endotracheal tube remains in place.

It will be appreciated that this may avoid the need to pass the intubation guide 110 over the tube fitting at the proximal end of the intubation tube 150 and the need to disconnect the proximal end of the endotracheal tube 150 which would undesirably interrupt ventilation of the subject using the endotracheal tube 150. This can also avoid the risk of unintentionally displacing the endotracheal tube 150 from its delivered position and potentially losing the secure airway. The intubation guide 110 will typically be designed so that the passageway 112 is fitted to the blade portion 142 of the intubation device 140 without having to build in an allowance for the tube fitting. As will be discussed further in due course, this can help to reduce leakage of ventilation gas around the blade portion 142 during the procedure.

In a similar manner, the ventilation mask 120 may be configured to be broken about the intubation port 124, such that the method may include breaking the ventilation mask 120 to allow the ventilation mask to be removed while the endotracheal tube remains in place.

In any event, in view of the above description of the method, it will be appreciated that the apparatus for use in such a ventilated intubation procedure will include the intubation guide 110 and the ventilation mask 120.

The intubation guide 110 includes an elongate body 111 defining a passageway 112 extending between a proximal opening 113 and a distal opening 114. The passageway 112 is particularly configured for receiving a blade portion 142 of an intubation device 140. The intubation guide is configured for insertion into a mouth 102 of the subject so that the proximal opening 113 is positioned in the mouth 102 and the distal opening 114 is positioned proximate to a pharynx 203 of the subject 100.

The elongate body 111 of the intubation guide 110 may be curved as depicted in the example of Figures 5A to 5C. Such a curved configuration may be provided so that the intubation guide 110 can better conform to the mouth 102 and airway anatomy of the subject in use. However, it is not essential that the intubation guide 110 be curved, and in alternative embodiments, the intubation guide 110 may be provided with a straight configuration.

The intubation guide 110 may be formed from different materials having different levels of flexibility depending on requirements. For instance, a straight intubation guide 110 may be formed from a relatively flexible material such that the intubation guide 110 may be allowed to at least partially deform in order to conform to the natural curvature of the mouth 102 and airway anatomy of the subject in use. On the other hand, a curved intubation guide 110 may be formed from a relatively inflexible material, where the curvature will need to be selected to conform to the subject's anatomy without relying on substantial deformation of the intubation guide 110.

It will be appreciated that the intubation guide 110 may serve to allow the blade portion 142 of the intubation device 140 to be inserted into the airway of the subject 100 without interference with the subject's tongue and other anatomical structures which may otherwise obstruct the insertion of the blade portion 142 in use. This is especially advantageous since the ventilation mask 120 may prevent direct visualisation during insertion of the blade portion 142. It should be understood that the intubation guide 110 will typically act to hold the tongue of the subject and typically to depress the tongue, i.e. to urge the tongue downwardly. This may depend on the particular shape and configuration of the intubation guide 110, including the curvature and flexibility as discussed above.

The ventilation mask 120 is for covering the mouth 102 of the subject 100 and includes a ventilation port 122 for connection to a ventilator 130, an intubation port 124 for receiving the blade portion 142 of the intubation device 140. The intubation port 124 is configured to align with the mouth 102 of the subject 100 to thereby allow the blade portion 142 to be inserted into the passageway 112 of the intubation guide 110 through the intubation port 124. Preferably, the intubation guide 110 and the ventilation mask 120 will be designed to suit the intubation device 140 and particularly its blade portion 142. The intubation device 140 and endotracheal tube 150 that are used in the procedure may not necessarily be provided with any specific adaptations for use in a ventilated endotracheal intubation. Accordingly, the intubation guide 110 and the ventilation mask 120 may be provided separately to the intubation device 140 and endotracheal tube 150, provided the correct type of intubation device 140 and respective blade portion 142 are selected for the intubation guide 110 and the ventilation mask 120.

Further optional features of the apparatus and their associated advantages will now be described.

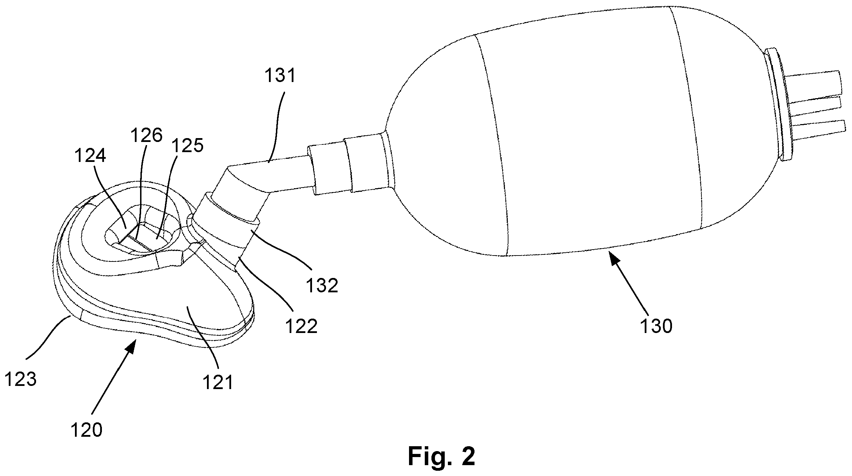

The ventilation mask 120 and the ventilator 130 are shown connected together in Figure 2, and further details of the ventilation mask 120 can be shown in the separate views of Figures 3A and 3B.

As mentioned above, in this example, a manual bag-valve type ventilator 130 is used and is connected to the ventilator port 122 of the ventilator mask 120 using a ventilator connector 132 at the end of a ventilator tube 131. The ventilator connection 132 will typically be a standard/universal connector type. In other examples, a separate ventilator unit may be connected to the ventilator port 122 by longer flexible tubing.

The basic construction of the ventilation mask 120 may be similar to conventional ventilation face masks. The ventilation mask 120 will typically have a contoured body 121 surrounded by a cushioned rim 123 that forms a seal on the subject's face around at least the subject's mouth, generally both the mouth and nose. Typically, the ventilation mask 120 will be formed from a suitable medical grade plastic material, preferably being transparent to allow visualisation of the mouth in use. However, the use of a transparent material may be unnecessary if a laryngoscope with a video capability is used.

The ventilator port 122 will typically be positioned away from the mouth of the user so that the intubation port 124 can be positioned over the mouth in use. For example, the ventilation mask 120 may be designed to cover the mouth and nose of the subject in a conventional manner, with the ventilation port 122 located proximate to a nose covering portion of the ventilation mask 120. As per the depicted example of the ventilation mask, the ventilation port 122 may be offset laterally relative to a central plane of the ventilation mask 120 that will be aligned with a sagittal plane of the subject 100 when the ventilation mask 120 covers the mouth 102 of the subject 100. This will locate the ventilation port 122 on one side of the subject's nose, and thus allow ventilation gas to be supplied proximate to the nose in use.

In some embodiments, the ventilation port 122 may be particularly configured to direct airflow towards nostrils of the subject's nose. This may help to ensure that ventilation gas is less likely to escape from the intubation port in the case of an imperfect closure or seal.

In some implementations, the ventilation mask 120 may include an internal partition for defining separate volumes proximate to the mouth and the nose of the subject. For example, the internal partition may be in the form of a membrane or barrier that extends laterally across the internal volume of the ventilation mask 120, being connected to the inner wall of the body 121, so that the internal volume is divided into a mouth volume around the mouth and a nose volume around the nose. The internal partition may be formed integrally with the body 121 or provided as a separate component attached to the inside of the body using suitable attachment techniques. The internal partition will have a free edge that may be configured to engage with the face of the subject between the mouth and the nose, which may provide a seal to prevent air communication between the two volumes. Accordingly, ventilation gas may be directed from the ventilation port 122 into the nose volume with a reduced risk of the ventilation gas escaping out of intubation port 124 before actually ventilating the subject.

Preferably, the ventilation mask 120 will be configured to allow the subject 100 to be ventilated using the ventilation mask 120 without allowing ventilation gas to escape from the intubation port 124 before and during inserting the blade portion 142 of the intubation device 140. In some implementations, this can be achieved by providing the ventilation mask 120 with a closure (not shown) for covering the intubation port 124, such that the closure may be kept in place during ventilation but removed prior to inserting the blade portion 142 through the intubation port 124. Following insertion, the blade portion 142 may be sufficient to substantially prevent the escape of gas from the intubation port. Once the intubation procedure is completed, ventilation can continue via the endotracheal tube. The closure may thus be provided in the form of a removable cap or plug which may be applied to the intubation port 124 as required using a range of different possible interfaces, such as a threaded connection, an interference fit, or the like. Alternatively, the closure may be provided in the form of a movable cover such as a hingedly attached barrier that can be pushed out of the way upon insertion of the blade portion 142.

In some implementations as shown in Figures 3A and 3B, the ventilation mask 120 may include a seal 125 covering the intubation port 124. The seal 125 may be configured to be normally in a closed position for sealing the intubation port 124 and may be moveable to an open position when the blade portion 142 of the intubation device 140 is inserted through the intubation port 124. Turning back to Figure 1C, it will be appreciated that, when the blade portion 142 of the intubation device 140 is first inserted into the intubation port 124, this may cause the seal 125 in the intubation port 124 to move from the closed position to the open position, to thereby permit the blade portion 142 to pass through the intubation port 124.

As mentioned above, the seal 125 may be moveable from a normally closed position (as shown in Figures 3A and 3B) to an open position when the blade portion 142 of the intubation device 140 is inserted through the intubation port 124 (as shown in Figures 4A and 4B). In some embodiments, the seal 125 may include at least one resilient membrane configured to deform in response to the blade portion 142 being urged against the seal 125, to thereby define an opening for receiving the blade portion 142.

In one example, the seal 125 may include two or more resilient membranes that are each supported around a respective part of a perimeter of the intubation port 124 and that each include a respective unsupported edge 126. The respective unsupported edges 126 may at least partially overlap in the closed position and separate to define the opening in the open position. Although overlapping edges 126 are preferred for more effective sealing, in some examples the edges 126 may be abutted in the closed position without any overlap.

In other examples, the seal 125 may include two or more resilient membranes that are supported around the perimeter of the intubation port 124. The respective aperture of each resilient membrane may differ from the apertures of other resilient membranes in shape, location or orientation. In other words, the seal 125 may include multiple fully supported membranes with apertures such as slits that do not align or overlap, to thereby provide an enhance sealing effect.

In another example, the seal 125 may include a single resilient membrane that is supported around a perimeter of the intubation port 124. The single resilient membrane may include an aperture that is substantially closed in the closed position and that stretches to define the opening in the open position. For instance, in some embodiments, the aperture may be a slit. In other embodiments, the aperture may be a pinhole, or may have any other geometry selected to allow a suitable opening to be formed for receiving the blade portion 142 of the intubation device 140. For instance, the aperture could be cross shaped or H shaped.

The at least one resilient membrane may be formed from any suitable membrane material, although typically a thin, flexible polymer material will be used. It may also be desirable to form the membranes from a transparent material as mentioned above for the body 121 of the ventilation mask 120.

It will be appreciated that, when the blade portion 142 is received in the ventilation port 122 as shown in Figures 4A and 4B, the seal 125 will be in the open configuration but may still provide a seal around the blade portion 142 to substantially prevent the escape of ventilation gas around the blade portion 142 during the intubation procedure. The degree of sealing or leakage will depend on the design of the seal and also the design of the blade portion 142. For instance, if the seal 125 is configured to stretch around the blade portion 142 in the open position this may form a substantially airtight seal even when the blade portion 142 extends through the ventilation port 122.

Some leakage still take place via the channel 401 that extends through the blade portion 142 to allow delivery of the endotracheal tube 150, especially in open channel designs as depicted. In some examples, the intubation device 140 may be designed to include a channel seal (not shown) as described in WO/2016/090435A1. Such a channel seal can be used to prevent the escape of gas from the ventilation port 122 along the channel 401. However, this is not essential, and in some intubation device 140 designs the endotracheal tube 150 may fit in the channel 401 so as to not provide a significant leakage path and the seal 125 may conform to the blade portion sufficiently well to prevent substantial leakage.

Moving on to the intubation guide 110, this will typically be provided separate from the ventilation mask 120 and moveable relative to the ventilation mask 120 in use. It will be appreciated that this will allow the intubation guide 110 to be move as the blade portion 142 of the intubation device 140 is inserted through the passageway 112 and specifically as the distal tip 143 is moved to a suitable position to allow the endotracheal tube 150 to be advanced through the larynx 104 into the trachea 105 of the subject. However, in some implementations, the intubation guide 110 may be formed integrally with the ventilation mask 120.

With regard to the detailed example of the intubation guide 110 shown in Figures 5A to 5C, it can be seen that the elongate body 111 may be defined as a thin walled body to define the passageway 112. The body 111 will typically be formed from a rigid, semi-rigid or flexible material such as a suitable medical grade plastic material. Whilst transparency may be a desirable quality for the ventilation mask 120, this is less important for the intubation guide 110, but still could be useful.

In this example, the intubation guide 110 includes a flange 115 surrounding the proximal opening 113. The flange 115 may be configured to prevent over-insertion of the intubation guide 110 by abutting the subject's mouth 102, to thereby ensure that the proximal opening 113 remains positioned outside the mouth. Although such a flange 115 is not essential, if a flange 115 is not provided then some other means of preventing over-insertion may be needed.

As mentioned above, it may be desirable to provide an intubation guide 110 that is configured to be broken along the passageway 112, to facilitate removal of the intubation guide 110 while the endotracheal tube 150 remains in place.

In this particular example, the body 111 of the intubation guide 110 includes two break lines 501 extending longitudinally along opposing sides of the passageway 112, to thereby allow the intubation guide 110 to be broken along the break lines 501. The break lines could be formed by providing a region of significantly thinner material compared to the material making the walls of the body 111, such that this thinner region would be breakable when opposing sides of the body 111 are forcibly pulled apart.

In this case, it will be seen that the break lines 501 are defined along a central plane of the intubation guide 110. These break lines 501 will be aligned with a sagittal plane of the subject in use. However, it should be appreciate that the use of break lines 501 in the depicted configuration is not essential, and the intubation guide 110 may include a different arrangement to allow it to be broken to facilitate its removal. For instance, a single break line 501 may be provided on one side of the passageway 112 and a hinge line may be defined on the opposite side of the passageway 112 so that the intubation guide 110 can be broken into two hingedly connected portions, which would nevertheless facilitate easier removal while the endotracheal tube 150 remains in place.

Since the intubation port 124 of the ventilation mask 120 and the passageway 112 of the intubation guide 110 must both receive the blade portion 142 of the intubation device 140, it should be appreciated that they will typically have similar cross sections. As can be seen in the examples, each of the intubation port 124 and the passageway 112 have generally square shaped cross sections with rounded corners, which matches the cross section shape of the blade portion 142 as shown in Figures 4A and 4B, and Figures 6A and 6B. However the shape may vary depending on the particular shape of the blade portion 142.

Accordingly, a shape of the intubation port 124 may be selected based on a cross section shape of the blade portion 142 of the intubation device 140, and a shape of the passageway may be selected based on a cross section shape of the blade portion 142 of the intubation device 140. As mentioned above, the intubation guide 110 may be curved, and if so, the curvature of the intubation guide 110 may be selected based on a curvature of the blade portion 142 of the intubation device 140.

The degree of curvature may also depend on a range of other factors including the flexibility of the material used to form the body 111 and the airway anatomy of the subject. For instance, as discussed above, the use of a more flexibly material may allow a straight or relatively uncurved body 111 to deform in use and conform to the blade portion 142 and/or the subject's airway. It should be appreciated that the curvature of the body 111 may be less critical if it is formed from a relatively flexible material. It should be noted, however, that there will be a practical limit to the flexibility of the intubation guide 110 to prevent its collapse upon insertion into the subject's mouth.

Furthermore, the use of a relatively more flexible material to form the body 111 of the intubation guide 110 may allow different sizes and shapes of the blade portion 142 to be accommodated, whereas the use of a relatively more rigid material may limit the range of blade types such that different intubation guides 120 may need to be selected for different sizes and shapes of the blade portion 142.

It should be noted that a variety of different shapes and sizes of intubation guides 110 and ventilation masks 120 may be provided to accommodate the range of different types, shapes and sizes of blades that may be used with the intubation device to suit patients having different ages, sizes and anatomical configurations. For instance, different intubation guides 110 and ventilation masks 120 may be provided for use with pediatric, adult or obese subjects, and will be selected to correspond with the selected blade for the particular subject.

However, some of the above described techniques, such as the use of flexible materials to form the intubation guide 110 or ventilation mask 120, may allow a single intubation guide 110 and ventilation mask 120 to be used with a range of different blades.

In some examples, the intubation port 124 of the intubation mask 120 may be deliberately constructed so that its size is smaller than a corresponding cross section size of the blade portion 142 of the intubation device 140. Similarly, the passageway 112 of the intubation guide 110 may be deliberately constructed so that its size is smaller than a corresponding cross section size of the blade portion 142 of the intubation device 140. Providing a smaller sized intubation port 124 and/or passageway 112 can ensure a snug fit which can help to seal against the escape of gases around the blade portion 142 at its interfaces with the intubation port 124 and passageway 112.

This sealing effect may be more pronounced if a flexible material is used to form the intubation guide 110 and/or the ventilation mask 120, since this may enable the intubation port 124 and/or passageway 112 to be sized significantly smaller than the corresponding cross section size of the blade portion 142 and stretch to accommodate the blade portion 142 in use. In this regard, it will be appreciated that at least one of the intubation guide 110 and the intubation port 124 may be configured to expand when receiving the blade portion 142. Forming the intubation guide 110 and the intubation port 124 from an expandable material can thus allow the blade portion 142 to be more tightly enclosed within the intubation guide 110 and/or the intubation port 124 in use.

In any event, it will be appreciated that the above described method and apparatus can allow ventilated intubation to be performed to reduce the risks associated with loss of ventilation during the procedure, whilst using a familiar intubation device.

Throughout this specification and claims which follow, unless the context requires otherwise, the word "comprise", and variations such as "comprises" or "comprising", will be understood to imply the inclusion of a stated integer or group of integers or steps but not the exclusion of any other integer or group of integers. As used herein and unless otherwise stated, the term "approximately" means ±20%.

It must be noted that, as used in the specification and the appended claims, the singular forms "a," "an," and "the" include plural referents unless the context clearly dictates otherwise. Thus, for example, reference to "a support" includes a plurality of supports. In this specification and in the claims that follow, reference will be made to a number of terms that shall be defined to have the following meanings unless a contrary intention is apparent.

It will of course be realised that whilst the above has been given by way of an illustrative example of this technology, all such and other modifications and variations hereto, as would be apparent to persons skilled in the art, are deemed to fall within the broad scope and ambit of this technology as is herein set forth.

## Claims

1. Apparatus (100) for use in an endotracheal intubation procedure, the apparatus including:
a) an intubation guide (110) including an elongate body (111) defining a passageway (112) extending between a proximal opening (113) and a distal opening (114), and the intubation guide (110) being configured for insertion into a mouth of the subject so that the proximal opening (113) is positioned proximate to the mouth of the subject and the distal opening (114) is positioned proximate to a pharynx of the subject; and
b) a ventilation mask (120) for covering the mouth of the subject, the ventilation mask (120) including:
i) a ventilation port (122) for connection to a ventilator (130); and
ii) an intubation port (124),
**characterised in that** the passageway (112) of the intubation guide (110) is configured for receiving a blade portion (142) of an intubation device (140), and the intubation port (124) is for receiving the blade portion (142) of the intubation device (140), the intubation port (124) being configured to align with the mouth of the subject to thereby allow the blade portion (142) to be inserted into the passageway (112) of the intubation guide (110) through the intubation port (124).

2. Apparatus (100) according to claim 1, wherein the ventilation mask (120) includes a seal (125) covering the intubation port (124), the seal (125) normally being in a closed position for sealing the intubation port (124) and being moveable to an open position when the blade portion (142) of the intubation device (140) is inserted through the intubation port (124).

3. Apparatus (100) according to claim 2, wherein the seal (125) includes at least one resilient membrane configured to deform in response to the blade portion (142) being urged against the seal (125), to thereby define an opening for receiving the blade portion (142).

4. Apparatus (100) according to claim 3, wherein at least one of:
a) the seal (125) includes a resilient membrane that is supported around a perimeter of the intubation port (124), the resilient membrane including an aperture that is substantially closed in the closed position and that stretches to define the opening in the open position, and optionally wherein the aperture is a slit;
b) the seal (125) includes two or more resilient membranes that are supported around the perimeter of the intubation port (124), each resilient membrane including a respective aperture that is substantially closed in the closed position and that stretches to define the opening in the open position, the respective aperture of each resilient membrane differing from the apertures of other resilient membranes in at least one of: shape; location; and orientation; and
c) the seal (125) includes two or more resilient membranes that are each supported around a respective part of a perimeter of the intubation port (124) and that each include a respective unsupported edge, the unsupported edges at least partially overlapping in the closed position and separating to define the opening in the open position.

5. Apparatus (100) according to any one of claims 1 to 4, wherein the ventilation mask (120) is for covering the mouth and a nose of the subject, and wherein at least one of:
a) the ventilation port (122) is located proximate to the nose covering portion of the ventilation mask (120);
b) the ventilation port (124) is configured to direct a flow of ventilation gas towards nostrils of the subject;
c) the ventilation mask (120) includes an internal partition for defining separate volumes proximate to the mouth and the nose of the subject; and
d) the ventilation port (122) is offset laterally relative to a central plane of the ventilation mask (120) that is aligned with a sagittal plane of the subject when the ventilation mask (120) covers the mouth of the subject.

6. Apparatus (100) according to any one of claims 1 to 5, wherein the ventilation mask (120) is configured to be broken about the intubation port (124).

7. Apparatus (100) according to any one of claims 1 to 6, wherein the intubation guide (110) is separate from the ventilation mask (120) and moveable relative to the ventilation mask (120) in use.

8. Apparatus (100) according to any one of claims 1 to 7, wherein the intubation guide (110) includes a flange (115) surrounding the proximal opening (113), and optionally wherein the flange (115) is configured to prevent over-insertion of the intubation guide (110) by abutting the subject's mouth to thereby ensure that the proximal opening (113) remains positioned outside the mouth.

9. Apparatus (100) according to any one of claims 1 to 8, wherein the intubation guide (110) is configured to be broken along the passageway (112), and optionally wherein the body (111) of the intubation guide (110) includes two break lines (501) extending longitudinally along opposing sides of the passageway (112), to thereby allow the intubation guide (110) to be broken along the break lines (501).

10. Apparatus (100) according to any one of claims 1 to 9, wherein the intubation port (124) of the ventilation mask (120) and the passageway (112) of the intubation guide (110) have similar cross sections.

11. Apparatus (100) according to any one of claims 1 to 10, wherein at least one of:
a) a shape of the intubation port (124) is selected based on a cross section shape of the blade portion (142) of the intubation device (140);
b) a size of the intubation port (124) is selected based on a cross section size of the blade portion (142) of the intubation device (140), and is optionally smaller than the cross section size of the blade portion (142) of the intubation device (140);
c) a shape of the passageway (112) is selected based on a cross section shape of the blade portion (142) of the intubation device (140); and
d) a size of the passageway (112) is selected based on a cross section size of the blade portion (142) of the intubation device (140), and is optionally smaller than the cross section size of the blade portion (142) of the intubation device (140).

12. Apparatus (100) according to any one of claims 1 to 11, wherein at least one of the intubation guide (110) and the ventilation mask (120) is formed from a flexible material.

13. Apparatus (100) according to any one of claims 1 to 12, wherein at least one of the intubation guide (110) and the intubation port (124) is configured to expand when receiving the blade portion (142).

14. Apparatus (100) according to any one of claims 1 to 13, wherein the intubation guide (110) is curved, and optionally wherein a curvature of the intubation guide (110) is selected based on a curvature of the blade portion (142) of the intubation device (140).

15. Apparatus (100) according to any one of claims 1 to 14, wherein the intubation guide (110) is configured to, in use, at least one of:
a) hold a tongue of the subject; and
b) depress the tongue.

## Patentansprüche

1. Gerät (100) zur Verwendung in einem endotrachealen Intubationsvorgang, wobei die Vorrichtung umfasst:
a) eine Intubationsführung (110), die einen länglichen Körper (111) umfasst, der einen Durchgang (112) definiert, der sich zwischen einer proximalen Öffnung (113) und einer distalen Öffnung (114) erstreckt, und wobei die Intubationsführung (110) zur Einführung in einen Mund des Patienten ausgestaltet ist, derart dass die proximale Öffnung (113) nahe an dem Mund des Patienten positioniert ist, und die distale Öffnung (114) nahe an einem Pharynx des Patienten positioniert ist; und
b) eine Beatmungsmaske (120) zum Bedecken des Mundes des Patienten, wobei die Beatmungsmaske (120) umfasst:
i) einen Beatmunganschluss (122) zur Verbindung mit einem Beatmungsgerät (130); und
ii) einen Intubationsanschluss (124),
**dadurch gekennzeichnet, dass** der Durchgang (112) der Intubationsführung (110) zum Aufnehmen eines Spatelabschnitts (142) einer Intubationsvorrichtung (140) ausgestaltet ist, und der Intubationanschluss (124) zum Aufnehmen des Spatelabschnitts (142) der Intubationsvorrichtung (140) bestimmt ist, wobei der Intubationsanschluss (124) dazu ausgestaltet ist, sich mit dem Mund des Patienten auszurichten, um dadurch das Einführen des Spatelabschnitts (142) durch den Intubationsanschluss (124) in den Durchgang (112) der Intubationsführung (110) zu ermöglichen.

2. Gerät (100) nach Anspruch 1, wobei die Beatmungsmaske (120) eine Dichtung (125) umfasst, welche den Intubationsanschluss (124) bedeckt, wobei die Dichtung (125) sich normalerweise in einer geschlossenen Position zum Dichten des Intubationsanschlusses (124) befindet und zu einer offenen Position beweglich ist, wenn der Spatelabschnitt (142) der Intubationsvorrichtung (140) durch den Intubationanschluss (124) eingeführt wird.

3. Gerät (100) nach Anspruch 2, wobei die Dichtung (125) mindestens eine nachgiebige Membran umfasst, die dazu ausgestaltet ist, sich als Reaktion darauf, dass der Spatelabschnitt (142) gegen die Dichtung (125) gedrängt wird, zu verformen, um dadurch eine Öffnung zum Aufnehmen des Spatelabschnitts (142) zu definieren.

4. Gerät (100) nach Anspruch 3, wobei mindestens eines von Folgendem zutrifft:
a) die Dichtung (125) umfasst eine nachgiebige Membran, die um einen Umkreis des Intubationsanschlusses (124) herum gestützt wird, wobei die nachgiebige Membran eine Apertur umfasst, die im Wesentlichen in der geschlossenen Position geschlossen ist und die sich streckt, um die Öffnung in der offenen Position zu definieren, und wobei die Apertur wahlweise ein Schlitz ist;
b) die Dichtung (125) umfasst zwei oder mehr nachgiebige Membranen, die um den Umkreis des Intubationsanschlusses (124) gestützt werden, wobei jede nachgiebige Membran eine jeweilige Apertur umfasst, die im Wesentlichen in der geschlossenen Position geschlossen ist und die sich streckt, um die Öffnung in der offenen Position zu definieren, wobei die jeweilige Apertur jeder nachgiebigen Membran sich von den Aperturen anderer nachgiebiger Membranen in mindestens einem von Folgendem unterscheidet: Form; Ort; und Ausrichtung; und
c) die Dichtung (125) umfasst zwei oder mehr nachgiebige Membranen, die jeweils um einen jeweiligen Teil eines Umkreises des Intubationsanschlusses (124) gestützt werden und die jeweils einen jeweiligen ungestützten Rand umfassen, wobei die ungestützten Ränder sich zumindest teilweise in der geschlossenen Position überlappen und sich trennen, um die Öffnung in der offenen Position zu definieren.

5. Gerät (100) nach einem der Ansprüche 1 bis 4, wobei die Beatmungsmaske (120) zum Bedecken des Mundes und einer Nase des Patienten bestimmt ist und wobei mindestens eines von Folgendem zutrifft:
a) der Beatmungsanschluss (122) befindet sich nahe an dem die Nase bedeckenden Abschnitt der Beatmungsmaske (120) ;
b) der Beatmungsanschluss (124) ist dazu ausgestaltet, eine Strömung von Beatmungsgas hin zu Nasenlöchern des Patienten zu richten;
c) die Beatmungsmaske (120) umfasst eine innere Abtrennung zum Definieren separater Volumina nahe an dem Mund und der Nase des Patienten; und
d) der Beatmungsanschluss (122) ist seitlich in Bezug auf eine mittlere Ebene der Beatmungsmaske (120) versetzt, die mit einer sagittalen Ebene des Patienten ausgerichtet ist, wenn die Beatmungsmaske (120) den Mund des Patienten bedeckt.

6. Gerät (100) nach einem der Ansprüche 1 bis 5, wobei die Beatmungsmaske (120) dazu ausgestaltet ist, um den Intubationsanschluss (124) herum aufgebrochen zu werden.

7. Gerät (100) nach einem der Ansprüche 1 bis 6, wobei die Intubationsführung (110) von der Beatmungsmaske (120) getrennt und bei der Verwendung in Bezug auf die Beatmungsmaske (120) beweglich ist.

8. Gerät (100) nach einem der Ansprüche 1 bis 7, wobei die Intubationsführung (110) einen Flansch (115) umfasst, der die proximale Öffnung (113) umgibt, und wobei wahlweise der Flansch (115) dazu ausgestaltet ist, Übereinführung der Intubationsführung (110) durch Anschlagen an den Mund des Patienten zu verhindern, um dadurch sicherzustellen, dass die proximale Öffnung (113) außerhalb des Mundes positioniert bleibt.

9. Gerät (100) nach einem der Ansprüche 1 bis 8, wobei die Intubationsführung (110) dazu ausgestaltet ist, entlang des Durchgangs (112) aufgebrochen zu werden, und wobei wahlweise der Körper (111) der Intubationsführung (110) zwei Bruchlinien (501) umfasst, die sich in Längsrichtung entlang gegenüberliegender Seiten des Durchgangs (112) erstrecken, um dadurch das Aufbrechen der Intubationsführung (110) entlang der Bruchlinien (501) zu erlauben.

10. Gerät (100) nach einem der Ansprüche 1 bis 9, wobei der Intubationsanschluss (124) der Beatmungsmaske (120) und der Durchgang (112) der Intubationsführung (110) ähnliche Querschnitte aufweisen.

11. Gerät (100) nach einem der Ansprüche 1 bis 10, wobei mindestens eines von Folgendem zutrifft:
a) eine Form des Intubationsanschlusses (124) wird basierend auf einer Querschnittsform des Spatelabschnitts (142) der Intubationsvorrichtung (140) ausgewählt;
b) eine Größe des Intubationsanschlusses (124) wird basierend auf einer Querschnittsgröße des Spatelabschnitts (142) der Intubationsvorrichtung (140) ausgewählt und ist wahlweise kleiner als die Querschnittsgröße des Spatelabschnitts (142) der Intubationsvorrichtung (140);
c) eine Form des Durchgangs (112) wird basierend auf einer Querschnittsform des Spatelabschnitts (142) der Intubationsvorrichtung (140) ausgewählt; und
d) eine Größe des Durchgangs (112) wird basierend auf einer Querschnittsgröße des Spatelabschnitts (142) der Intubationsvorrichtung (140) ausgewählt und ist wahlweise kleiner als die Querschnittsgröße des Spatelabschnitts (142) der Intubationsvorrichtung (140).

12. Gerät (100) nach einem der Ansprüche 1 bis 11, wobei mindestens eine von der Intubationsführung (110) und der Beatmungsmaske (120) aus einem flexiblen Material gebildet ist.

13. Gerät (100) nach einem der Ansprüche 1 bis 12, wobei mindestens eines von der Intubationsführung (110) und dem Intubationsanschluss (124) dazu ausgestaltet ist, sich aufzuweiten, wenn sie bzw. er den Spatelabschnitt (142) aufnimmt.

14. Gerät (100) nach einem der Ansprüche 1 bis 13, wobei die Intubationsführung (110) gekrümmt ist und wobei wahlweise eine Krümmung der Intubationsführung (110) basierend auf einer Krümmung des Spatelabschnitts (142) der Intubationsvorrichtung (140) ausgewählt wird.

15. Gerät (100) nach einem der Ansprüche 1 bis 14, wobei die Intubationsführung (110) bei der Verwendung für mindestens eines von Folgendem ausgestaltet ist:
a) Halten einer Zunge des Patienten; und
b) Herunterdrücken der Zunge.

## Revendications

1. Appareil (100) destiné à être utilisé dans une procédure d'intubation endotrachéale, l'appareil comprenant :
a) un guide d'intubation (110) comprenant un corps allongé (111) définissant un passage (112) s'étendant entre une ouverture proximale (113) et une ouverture distale (114), et le guide d'intubation (110) étant configuré pour être inséré dans la bouche du sujet de telle sorte que l'ouverture proximale (113) soit positionnée à proximité de la bouche du sujet et que l'ouverture distale (114) soit positionnée à proximité du pharynx du sujet ; et
b) un masque de ventilation (120) destiné à recouvrir la bouche du sujet, le masque de ventilation (120) comprenant :
i) un orifice de ventilation (122) destiné à être raccordé à un ventilateur (130) ; et
ii) un orifice d'intubation (124),
**caractérisé en ce que** le passage (112) du guide d'intubation (110) est configuré pour recevoir une partie de lame (142) d'un dispositif d'intubation (140), et l'orifice d'intubation (124) est destiné à recevoir la partie de lame (142) du dispositif d'intubation (140), l'orifice d'intubation (124) étant configuré pour s'aligner avec la bouche du sujet afin de permettre ainsi à la partie lame (142) d'être insérée dans le passage (112) du guide d'intubation (110) à travers l'orifice d'intubation (124).

2. Appareil (100) selon la revendication 1, dans lequel le masque de ventilation (120) comprend un joint d'étanchéité (125) recouvrant l'orifice d'intubation (124), le joint (125) étant normalement dans une position fermée pour sceller l'orifice d'intubation (124) et pouvant être déplacé vers une position ouverte lorsque la partie lame (142) du dispositif d'intubation (140) est insérée à travers l'orifice d'intubation (124).

3. Appareil (100) selon la revendication 2, dans lequel le joint d'étanchéité (125) comprend au moins une membrane élastique configurée pour se déformer en réponse à la pression exercée par la partie lame (142) contre le joint d'étanchéité (125), afin de définir ainsi une ouverture pour recevoir la partie lame (142).

4. Appareil (100) selon la revendication 3, dans lequel au moins l'un des éléments suivants :
a) le joint d'étanchéité (125) comprend une membrane élastique qui est supportée autour du périmètre de l'orifice d'intubation (124), la membrane élastique comprenant une ouverture qui est sensiblement fermée en position fermée et qui s'étire pour définir l'ouverture en position ouverte, et éventuellement dans lequel l'ouverture est une fente ;
b) le joint (125) comprend deux ou plusieurs membranes élastiques qui sont supportées autour du périmètre de l'orifice d'intubation (124), chaque membrane élastique comprenant une ouverture respective qui est sensiblement fermée en position fermée et qui s'étire pour définir l'ouverture en position ouverte, l'ouverture respective de chaque membrane élastique différent des ouvertures des autres membranes élastiques par au moins l'un des éléments suivants : la forme ; l'emplacement ; et l'orientation ; et
c) le joint (125) comprend deux membranes élastiques ou plus qui sont chacune supportées autour d'une partie respective d'un périmètre de l'orifice d'intubation (124) et qui comprennent chacune un bord non supporté respectif, les bords non supportés se chevauchant au moins partiellement dans la position fermée et se séparant pour définir l'ouverture dans la position ouverte.

5. Appareil (100) selon l'une quelconque des revendications 1 à 4, dans lequel le masque de ventilation (120) est destiné à recouvrir la bouche et le nez du sujet, et dans lequel au moins l'un des éléments suivants :
a) l'orifice de ventilation (122) est situé à proximité de la partie du masque de ventilation (120) qui recouvre le nez ;
b) l'orifice de ventilation (124) est configuré pour diriger un flux de gaz de ventilation vers les narines du sujet ;
c) le masque de ventilation (120) comprend une cloison interne pour définir des volumes séparés à proximité de la bouche et du nez du sujet ; et
d) l'orifice de ventilation (122) est décalé latéralement par rapport à un plan central du masque de ventilation (120) qui est aligné avec un plan sagittal du sujet lorsque le masque de ventilation (120) recouvre la bouche du sujet.

6. Appareil (100) selon l'une quelconque des revendications 1 à 5, dans lequel le masque de ventilation (120) est configuré pour être brisé au niveau de l'orifice d'intubation (124).

7. Appareil (100) selon l'une quelconque des revendications 1 à 6, dans lequel le guide d'intubation (110) est séparé du masque de ventilation (120) et mobile par rapport au masque de ventilation (120) lors de l'utilisation.

8. Appareil (100) selon l'une quelconque des revendications 1 à 7, dans lequel le guide d'intubation (110) comprend une bride (115) entourant l'ouverture proximale (113), et, en option, dans lequel la bride (115) est configurée pour empêcher une insertion excessive du guide d'intubation (110) en venant en butée contre la bouche du sujet afin de garantir que l'ouverture proximale (113) reste positionnée à l'extérieur de la bouche.

9. Appareil (100) selon l'une quelconque des revendications 1 à 8, dans lequel le guide d'intubation (110) est configuré pour être cassé le long du passage (112), et, en option, dans lequel le corps (111) du guide d'intubation (110) comprend deux lignes de rupture (501) s'étendant longitudinalement le long des côtés opposés du passage (112), afin de permettre au guide d'intubation (110) d'être cassé le long des lignes de rupture (501).

10. Appareil (100) selon l'une quelconque des revendications 1 à 9, dans lequel l'orifice d'intubation (124) du masque de ventilation (120) et le passage (112) du guide d'intubation (110) ont des sections transversales similaires.

11. Appareil (100) selon l'une quelconque des revendications 1 à 10, dans lequel au moins l'un des éléments suivants :
a) une forme de l'orifice d'intubation (124) est choisie en fonction d'une forme de section transversale de la partie lame (142) du dispositif d'intubation (140) ;
b) la taille de l'orifice d'intubation (124) est choisie en fonction de la taille de la section transversale de la partie lame (142) du dispositif d'intubation (140) et est éventuellement plus petite que la taille de la section transversale de la partie lame (142) du dispositif d'intubation (140) ;
c) la forme du passage (112) est choisie en fonction de la forme de la section transversale de la partie lame (142) du dispositif d'intubation (140) ; et
d) une taille du passage (112) est choisie en fonction d'une taille de section transversale de la partie lame (142) du dispositif d'intubation (140), et est éventuellement plus petite que la taille de section transversale de la partie lame (142) du dispositif d'intubation (140).

12. Appareil (100) selon l'une quelconque des revendications 1 à 11, dans lequel au moins l'un parmi le guide d'intubation (110) et le masque de ventilation (120) est formé à partir d'un matériau flexible.

13. Appareil (100) selon l'une quelconque des revendications 1 à 12, dans lequel au moins l'un parmi le guide d'intubation (110) et l'orifice d'intubation (124) est configuré pour se dilater lors de la réception de la partie lame (142).

14. Appareil (100) selon l'une quelconque des revendications 1 à 13, dans lequel le guide d'intubation (110) est courbé, et dans lequel, en option, la courbure du guide d'intubation (110) est choisie en fonction de la courbure de la partie lame (142) du dispositif d'intubation (140).

15. Appareil (100) selon l'une quelconque des revendications 1 à 14, dans lequel le guide d'intubation (110) est configuré pour, lors de l'utilisation, au moins l'une des opérations suivantes :
a) maintenir la langue du sujet ; et
b) appuyer sur la langue.
